# EUROPEAN PATENT SPECIFICATION

(11) **EP 1 962 856 B1**
(45) Date of publication and mention of the grant of the patent: **20.10.2010**
(21) Application number: 06829511.2
(22) Date of filing: 12.12.2006
(51) Int. Cl.: A61K 31/565, A61K 31/566, A61K 31/568, A61K 31/5685, A61K 31/57, C07J 1/00, A61P 25/00, A61P 25/28, A61P 9/10

(54) **MEDICAMENT BASED ON A MONOESTER OF STEROIDS WITH LONG CHAIN FATTY ACIDS**
MEDIKAMENT AUF BASIS EINES MONOESTERS VON STEROIDEN MIT LANGKETTIGEN FETTSÄUREN
MEDICAMENT A BASE DE MONOESTER DE STEROIDES AVEC DES ACIDES GRAS A LONGUE CHAINE

(30) Priority: 13.12.2005 IT MI20052377
(43) Date of publication of application: 03.09.2008
(73) Proprietor: Petroni, Anna, 20090 Segrate (IT)
(72) Inventor: Petroni, Anna, 20090 Segrate (IT)
(74) Representative: Cattaneo, Elisabetta
(86) International application number: PCT/EP2006/011925
(87) International publication number: WO 2007/068434

(56) References cited:
- EP-A1- 0 606 012
- WO-A-95/12402
- WO-A1-03/011300
- GB-A- 455 264
- US-A1- 2 192 935
- US-A1- 4 147 783
- K. MIESCHER ET AL.: "Weitere Untersuchungen über die Wirkungsverstärkung männlicher Sexualhormone durch Veresterung" BIOCHEM Z., vol. 294, 1937, pages 39-60, XP008076314
- WATANUKI M ET AL: "Stimulation of uptake of cholesteryl esters into adrenal tumor cells by ACTH and other agents" FEBS LETTERS 1979 NETHERLANDS, vol. 101, no. 2, 1979, pages 239-243, XP008076310
- RAJU ET AL: "ANDROSTERONE LONG CHAIN FATTY ACID ESTERS IN HUMAN BREAST CYST FLUID" JOURNAL OF CLINICAL ENDOCRINOLOGY AND METABOLISM, ENDOCRINE SOCIETY, CHEVY CHASE, MD, US, vol. 60, 1985, pages 940-946, XP008076295 ISSN: 0021-972X

## Description

The present invention concerns a monoester of steroids with either a saturated or an unsaturated C₁₆-C₂₄ fatty acid for use as a medicament. Particularly the present invention is related to a medicament capable to reduce the accumulation of cholesterol esters, by reducing the ACAT enzyme (acyl-coenzyme A: cholesterol acetyltransferase) effect. Advantageously the invention is also related to a new monoester of the androgen, 5α-androstan-3α,17β-diol (briefly named 3α-diol) with either a saturated or an unsaturated C₁₈-C₂₄ fatty acid.

The ACAT enzyme is responsible for the intracellular esterification of cholesterol. The intracellular accumulation of cholesterol esters, particularly cholesterol esters with saturated fatty acids is considered to be a characteristic sign of arteriosclerosis ("CL277,082: A novel inhibitor of ACAT-catalyzed cholesterol esterification and cholesterol absorption" Journal of Lipid Research, volume 30, 1989). Recently an association between the ACAT enzyme and Alzheimer's disease has been also suggested, because an effect of cholesterol metabolism on amyloid plaques development has been supposed (*New way of controlling cholesterol may help treat Alzheimer,* Massachusetts General Hospital, published in October 14, 2004).

The inclusion of cholesterol esterified with very long chain saturated fatty acids in the adrenal gland cortex and in testicles is also reported as evidence of the presence of a disease called adrenoleucodistrophy. Specifically adrenoleucodistrophy is a serious hereditary chromosome X-linked disease, by which there is an abnormal accumulation of very long chain saturated fatty acids in plasma and tissues, due to a reduced peroxisomal beta-oxidation.

Metabolic pathways of lipid and steroid metabolism are extremely complex and often correlated, by way of illustration, all steroid hormones, in humans, derive from cholesterol ("Williams textbook of Endocrinology", IX edition, chapter 12, page 521, Wilson, Foster, Kronenberg, Larsen publishers).

The alteration of lipid and steroid mechanism, with increased concentration of cholesterol esters, appears therefore an evidence for the indicated pathologies. Therefore a use of selective inhibitors for ACAT enzyme has been suggested in order to control the accumulation of cellular and circulating cholesterol esters ("The pharmacological basis of therapeutics", Goodman and Gilman's, edition X, chapter 36).

In regard to artheriosclerotic plaques, the ACAT inhibitor CL 277,082 (N-(2,4-difluorophenyl)-N-(4-neopentylbenzyl)-N-(n-heptyl)urea) turned out to be very strong and specific in inhibiting the ACAT activity ("Association between acyl-coenzyme A: cholesterol acyltrasferase gene and risk for Alzheimer's disease in Chinese", Zhao FG. Et al, Department of Neurology, First hospital of Peking University, Neuroscience Lett, July 22 2005; *"*On the mechanism by which an ACAT inhibitor (CL 277,082) influences plasma lipoproteins in the rat" Balasubramaniam S et al, Artherosclerosis, 1990 May; 82 (1-2): 1-5).

In order to treat adrenoleucodystrophy, dihydrotestosterone and the androgen 5α-androstan-3α,17β-diol (3α-diol) have been proposed as agents for controlling the metabolism of long chain saturated fatty acids (VLCFA), being capable to increase beta-oxidation and to reduce cholesterol esterification processes in patients suffering from the disease ("Effects of the testosterone metabolite diydrotestosterone and 5α-androstan-3α,17β-diol on very long chain fatty acid metabolism in X-adrenoleukodystrophic fibroblasts" A. Petroni et al, Life Sciences 73 (2003) 1567-1575 Ed. Elsevier).

Such a study has not provided a potential pharmacological therapy. The androgens as such have been used in the therapy of adrenoleucodystrophy but they didn't have a beneficial effect as reported by Maris (*"*X-linked adrenoleukodystrophy presenting as neurologically pure familial spastic paraparesis" T.Maris et al, Neurology 45 (1995) 1101-1104).

In order to treat adrenoleucodystrophy a use of erucic acid (C22:1), which is administered in form of triglyceride, has been also proposed ("Dietary Management of X-linked adrenoleukodystrophy" Hugo W. Moser et al, Annual Review of Nutrition, Vol. 15: 379-397, publication date July 1995). Such an acid resulted to be only partially effective in vitro and little effective in vivo because it failed to cross the hematoencephalic barrier.

It has been surprisingly found that some esters of steroids with either saturated or unsaturated C₁₆-C₂₄ fatty acids are capable to intervene in alteration of the lipid and steroid mechanisms, particularly by reducing the accumulation of cholesterol esters.

An object of the present invention is therefore to provide a medicament which is capable to treat diseases that show abnormal accumulation of cellular and circulating cholesterol esters.

An object of the present invention is therefore to provide also a medicament for the treatment of adrenoleucodystrophy, Alzheimer's disease and the treatment of arteriosclerosis.

Such objects have been achieved by providing a family of monoesters for use as a medicament as recited in claim 1.

Specifically the present invention is related to a medicament based on a monoester of a steroid of formula I 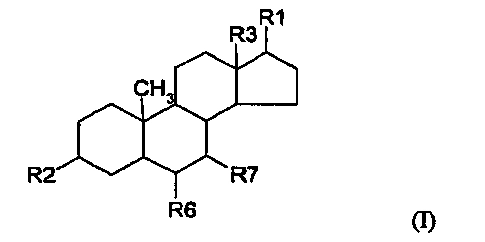 wherein
R1 and R2 are independently -OH or =O;
R3 is -CH₃;
R6 and R7 are independently -H or -OH, with either saturated or unsaturated C₁₈-C₂₄ fatty acid.

The following monoesters as medicament are also described, but do not form part of the invention; a steroid of formula II 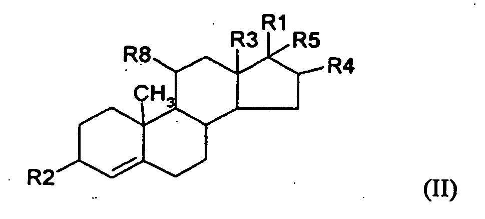 wherein
R1 is selected from the group consisting of =O,-OH,-COCH₃, and -
COCH₂OH;
R2 is =O;
R3 and R4 are independently -H, -CH₃, -OH, -CH₂OH or -CHO;
R5 is optionally present and it is -H or -OH;
R8 is -H or -OH;
or a steroid of formula III 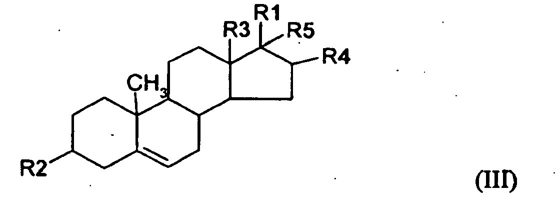 wherein
R1 is selected from the group consisting of =O and -COCH₃, R2 is -OH;
R3 and R4 are independently -H, -CH₃ or -OH;
R5 is optionally present and it is -H or -OH;
or a steroid of formula IV: 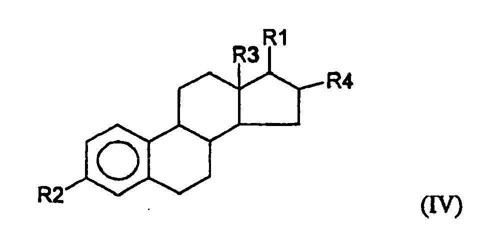 wherein
R1 is selected from the group consisting of -OH and =O;
R2 is -OH;
R3 and R4 are independently -H, -CH₃ or -OH;
with either a saturated or an unsaturated C₁₆-C₂₄ fatty acid.

Advantageously the invention concerns a monoester of a steroid having formula I with a C₁₈-C₂₂ fatty acid, preferably selected from the group consisting of palmitic acid, palmitoleic acid, oleic acid, stearic acid, elaidic acid, vaccenic acid, linoleic acid, conjugated linoleic acid, linolenic acid, α-linolenic acid, γ-linolenic acid, di-homo-gamma linolenic acid, eleostearic acid, arachidonic acid (eicosatetraenoic acid), adrenic acid, erucic acid, nervonic acid, docosapentaenoic acid, eicosatetraenoic acid, eicosapentaenoic acid as a medicament.

Preferably the fatty acid is an acid selected from the group consisting of linoleic acid, conjugated linoleic acid, linolenic acid, arachidonic acid and isomers thereof. More preferably it is linoleic acid (cis,cis-9,12-octadecadienoic acid) (18:2, n-6) or an isomer of conjugated linoleic acid. Among the isomers of conjugated linoleic acid (CLA), linoleic acid c9t11, namely cis,trans-9,11-octadecadienoic acid and linoleic acid t10c12, namely trans,cis-10,12-octadecadienoic acid may be mentioned. Among the derivatives of coniugated linoleic acid, cis,cis,trans-6,9,11-octadecatrienoic acid (18:3), cis,cis,trans-8,11,13-eicosatrienoic acid (20:3), cis,cis,trans-8,11,13-eicosatetraenoic acid (20:4) may be mentioned.

Among the derivatives of linolenic acid, gamma-linolenic acid (cis,cis,cis-6,9,12-octadecatrienoic acid) may be mentioned, among the derivatives of alfa-linolenic acid (cis,cis,cis-9,12,15-octadecatrienoic acid), eicosapentaenoic acid (cis,cis,cis,cis-5,8,11,14,17-eicosapentaenoic acid) may be mentioned.

The steroid of formula I of the present invention will be preferably selected from the group consisting of 5α-androstan-17β-ol-3-one (DHT), 5α-androstan-3α,17β-diol (3α-diol), 5α-androstan-3β,17β-diol (3β-diol), 5α-androstan-3β,6α,17β-triol (6α-triol), 5α-androstan-3β, 7β,17β-triol (7β-triol), 5α-androstan-3β,7α,17β-triol (7α-triol), 5α-androstan-3,17-dione (androstandione), 5α-androstan-3α-ol-17-one (androsterone), the steroid of formula II selected from the group consisting of testosterone, androstenedione, 16a-hydroxyandrostenedione, progesterone, 17α-hydroxyprogesterone, deoxycorticosterone, 11-deoxycortisol, cortisol, corticosterone, 18-hydroxycorticosterone, aldosterone; the steroid of formula III selected from the group consisting of dehydroepiandrosterone, 17α-hydroxypregnenolone, pregnenolone; and the steroid of formula IV selected from the group consisting of estrone, estradiol, estriol are also described, but not claimed.

It is also described a monoester of a steroid of formula V 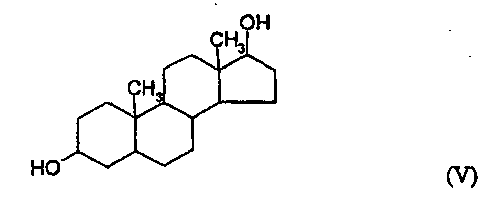 with a C₁₆-C₂₄ fatty acid. Preferably, the steroid of Formula V is 5α-androstan-3α,17β-diol (3α-diol) or 5α-androstan-3β,17β-diol (3β-diol). The invention concerns a monoester of the steroid of 5α-androstan-3α,17β-diol(3α-diol) with a C₁₈-C₂₄ fatty acid.

Such a monoester with the androgen 3α-diol or 3β-diol will be preferably an ester composed by a fatty acid selected from the group consisting of linoleic acid, conjugated linoleic acid, linolenic acid, arachidonic acid and isomers thereof, even more preferably it is an ester with linoleic acid (cis,cis-9,12-octadecadienoic acid (18:2, n-6)).

Preferably, the monoester of the invention is formed between the steroid and the fatty acid at either the position 3 or the position 17 of the steroid, the structure of which is usually numbered in the art as follows:

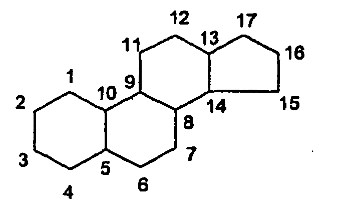

The monoester family of the invention may be used for the manufacture of a medicament for reducing the accumulation of cellular and circulating cholesterol esters as recited in claim 14, being said disease selected from the group consisting of adrenoleucodystrophy, Alzheimer's disease and arteriosclerosis.

Without wishing to be bound by any theory, as the ACAT enzyme plays a key role in the metabolism of fatty acids by forming cholesterol esters, it is believed that the reduction of the accumulation of cellular and circulating cholesterol esters can be achieved by reducing the activity of ACAT enzyme.

Thus, the medicament according to the invention is capable to perform its action in pathologies, in which an accumulation of cholesterol esters is seen and in which the ACAT enzyme plays a key role, such as adrenoleucodystrophy, Alzheimer's disease and arteriosclerosis.

Monoesters according to the invention may be prepared by organic chemistry synthesis methods, well known to those skilled in the art, which include the esterification reaction of the steroid with the carboxy group of the fatty acid.

Monoesters according to the invention may be also added with a pharmaceutically acceptable carrier for the manufacture of pharmaceutical compositions.

The invention will be now disclosed with reference to some examples of biochemical preparation and to an example of effectiveness on the inhibition of ACAT enzyme. Such examples are given by way of illustration of the invention.

### Example 1:

### Preparation of the monoester of the androgen 5α-androstan-3α,17β1-diol (3α-diol) and of the linoleic acid (LA) (cis,cis-9,12-octadecadienoic acid) (18:2, n-6)

For the preparation of the ester according to the invention, rat liver microsomes were used, which have an enzymatic system enabling the esterification of the carboxy group of fatty acids (in this case LA) with -OH group in 3 of steroids, in this case 3α-diol.

### - Preparation of rat liver microsomes

Microsomes were obtained by following the method by Einarsson et al., 1989.

The following buffers were prepared separately:
Tris-HCl buffer: pH 7.4, 50 mM, 500 ml with 50mM Tris buffer, 0.3 M Sucrose, 1 mM EDTANa₂ and protease inhibitors (PMSF phenyl methyl sulphonyl fluoride (1mM) and leupeptin (50 micromolar))
Potassium phosphate buffer: 0.1 M of potassium phosphate buffer pH 7.4, volume: 500 ml with 1 mM EDTA.

Rat liver was taken and weighed (9.56g), then perfused with physiological saline. "Potter" (glass system with pestle) was used to allow a gentle homogenation of the product obtained from perfusion with 50 ml of Tris-HCl buffer, pH 7.4.

Therefore, the solution was centrifuged at 20,000 revolutions for 15 min at 4°C. Then the surnatant was taken and centrifuged with ultracentrifuge at 100,000 revolutions for 1 hour (40,000 rpm).

The obtained pellet, consisting of microsomes, was then resuspended in 5 ml of potassium phosphate buffer, and subdivided in aliquots, that were freezed at -20°C, to be used in the different experiments.

One of these aliquots was used for protein assay in order to have a valid quantitative reference parameter in the different experiments. A volume of 250 microliters was used to which 500 microliters of soda were added. The solution was left to room temperature and the assay was performed according to classic procedure of Lowry et al., 1951.

The aliquot showed to have a protein concentration of 13.2 micrograms/microliter.

### - Synthesis of the ester of 3α-diol with linoleic acid (LA) (cis,cis-9,12-octadecadienoic acid) (18:2, n-6).

Synthesis of the ester was performed in duplicate. Two samples were then prepared (SAMPLE 1 and SAMPLE 2). The two samples were incubated with equal reagents in the same experimental conditions in order to compare the method reproducibility. Each sample contained 5 microliters of said microsomal preparation corresponding to 66 micrograms of proteins.

The solution A was prepared:
MOPS (400mM), MgCl₂ (50mM), KCl (50mM), CoASH (1mM), ATP (100mM), α-cyclodextrin (1 mg in 50 microliters H₂O per sample) and biphosphate buffer (42 microliters/sample).

In the two samples H₂O was added in order to have a final volume of 500 microliters per sample.

The following labelled compound was used:
androstan-3α,17β-diol,5α-(9,11-3H(N))(3α-diol-3H) (mother liquor) conc. 1mCi/ml in ethanol
specific activity 40 Ci/mmol
supplied by PerkinElmer Life Sciences, Inc.

In the two above samples the following:
linoleic acid: 72 microM (10.1 micrograms, 0.036 micromoles/sample) 3α-diol-3H: 0.1 microM (2microCi/2 microliters)
3α-diol: 72 microM (10.1 micrograms, 0.036 micromoles/sample) were incubated respectively.

The samples have been therefore incubated in said solution A for 10 min at 37 °C.

The incubation was therefore blocked for both samples with 2.5 ml of Dole's reagent (consisting of isopropilic alcohol 40 ml, heptane 10 ml, 1N H₂SO₄ 1 ml), 0.9 ml of H₂O, 1.5 ml of heptane.

After stirring, the separation in two phases was achieved. The upper phase was transferred in a tube. The lower phase was extracted twice more with 1.5 ml of heptane and the two upper phases obtained were joined with the first one in a single tube. The content was dried and taken-up with 1 ml of chloroform:methanol 2:1.

An aliquot per sample was then taken which was used for the radiation count with scintillation liquid (Ultima Gold).

The count was performed according to classic methods as reported in "Radioisotopes in Biology, a practical approach", published by R.J. Slater, Oxford University Press, 1990; "Effects of simvastatin on the metabolism of polyunsaturated fatty acids and on glycerolipid, cholesterol and de novo lipid synthesis in THP-1 cells" Risè et al. Journal Lipid Research, volume 38, 1299-1307,1997.

On the basis of the obtained radioactivity, the following ester concentration of LA with 3α-diol resulted:
SAMPLE 1: 15.17 microM (0.015166 micromoles, 8.38 micrograms)
SAMPLE 2: 15.02 microM (0.015016 micromoles, 8.33 micrograms)

### Example 2:

### Preparation of the ester of the 3α-diol with conjugated linoleic acid (CLA), isomer C9t11

The example 1 was repeated in order to obtain the monoester of the 3α-diol with the isomer of the linoleic acid (c9t11) starting from:
coniugated linoleic acid : 90 microM
3α-diol-3H: 0.1 microM (2microCi/2 microliters)
3α-diol: 90 microM

The following results were obtained:
Sample 1 : 18.01 microM
Sample 2 : 19.6 microM

### Example 3:

### Preparation of the monoester of the androgen 5α-androstan-3α,17β-diol (3α-diol) and of the linoleic acid (LA) (cis,cis-9,12-octadecadienoic acid) (18:2, n-6) by means of double labelling

Rat liver microsomes obtained according to the method disclosed in example 1 were used.

The synthesis was performed in two steps.

In the first step the linoleic acid (cis,cis-9,12-octadecadienoic acid) (18:2, n-6) was incubated in 60 micrograms of microsomes.

Specifically a solution containing MOPS (400mM), MgCl₂ (50mM), KCl (50mM), CoASH (1mM), ATP (100mM), α-cyclodextrin (1 mg in 50 microliters H₂O per sample) and biphosphate buffer (42 microliters/sample) was prepared.

In the sample of microsomes, H₂O was added in order to have a final volume of 500 microliters. Therefore linoleic acid (C18:0) 72 microM (0.036 micromolar) was incubated with 2 microCi of ¹⁴C-labelled linoleic acid (specific activity 55 mCi/mmoles, 5.5 microCi/0.1 micromol Amersham, Bioscience, UK).

Then incubation for 10 min at 37°C and subsequent extraction with Dole's reagent have been performed as disclosed in example 1. The extract was dried and taken-up again in liquid. An aliquot of liquid was counted in a beta counter.

In the second step of the preparation, said extract was taken-up again in 1 ml of water, to which BSA 1mg/ml in phosphate buffer, EDTA (200 microliters), microsomes 60 micrograms, 3α-diol-3H (0.1 microM (2microCi/2 microliters), 3α-diol 72 microM (10.1 micrograms, 0.036 micromoles/sample) were added. EDTA phosphate buffer was also added in order to have a final volume of 2000 microliters. The mixture has been incubated for 5 min at 37°C. An aliquot was then subjected to TLC with use of a standard in order to separate the ester and the ester-related band was transferred in a tube by extraction and subjected to radiation count in double labelling (labelling of the androgen and of the linoleic acid). Separate reading of two isotopes was performed: respectively ³H for 3α-diol and ¹⁴C for linoleic acid. The reading was automatically done by the beta counter instrument. The count was done according to classic methods as reported in "Radioisotopes in Biology, a practical approach", published by R.J. Slater, Oxford University Press, 1990; "Effects of simvastatin on the metabolism of polyunsaturated fatty acids and on glycerolipid, cholesterol, and de novo lipid synthesis in THP-1 cells" Risè et al. Journal Lipid Research, volume 38, 1299-1307,1997.

On the basis of obtained radioactivity, the following ester concentration resulted: 4.5 microM.

### Example 4:

### Preparation of the monoester of the androgen 5α-androstan-3α,17β-diol (3α-diol) and of the linoleic acid (LA) (cis,cis-9,12-octadecadienoic acid) (18:2, n-6) with execution of purification step

The example 1 was repeated both for the preparation of rat liver microsomes and the synthesis of the ester until the aliquots were taken-up for the radiation count with scintillation liquid (Ultima Gold).

Before performing the count, the ester-containing aliquots were purified using thin layer chromatography (TLC).

Two silica gel 60 TLC plates (20 x 20 cm) (Merck) were used for each aliquot, taken-up from the samples respectively. The plates have previously been heat-activated at 120 °C for 30 min, to remove dampness, which would have influenced the run of the compounds with TLC during different periods of the year. The plates were also cooled in an air-free glass system before use.

The two samples were run on the two plates using the following solvents: hexane:diethylether:acetic acid in amounts equivalent to 70:30:1.5.

The samples were run in the same chromatographic conditions, by using linoleic acid and 3α-diol as standards, which served to verify the reproducibility of the chromatography.

At the end of the run the plates were subjected to a nitrogen flow in order to remove the solvents and highlight the standard-corresponding bands and those produced by the two samples. The bands similar to standard bands resulted to be the reagents, linoleic acid and androstan, and the remaining to the formed ester. The silica corresponding to the ester bands of the two plates was then taken-up and put in two tubes respectively. The content of each tube corresponded to one band, which was extracted with the following solvents: methanol 5 ml, chloroform 2.5 ml. After stirring in vortex, further 2.5 ml of chloroform were used for the extraction, to which a further stirring followed and then an addition of 2.5 ml of H₂O, for 2 hours at -20°C in order to separate the phases. Therefore, the lower phases of the extraction from each tube were kept, which were dried and resuspended separately with 1 ml of chloroform:methanol (2: 1). An aliquot per sample was then taken-up and it was subjected to count by beta counter.

On the basis of obtained radioactivity, the following LA ester with 3α-diol concentration resulted:
SAMPLE 1: 2.98 microM (0.00297 micromoles, 1.65 micrograms)
SAMPLE 2: 3.64 microM (0.00364 micromoles, 2.01 micrograms)

### Example 5:

### Identification by mass spectrometry

The esters of linoleic acid (LA) (C18:2, n-6) with the androgen 5α-androstan-3α,17β-diol (3α-diol) obtained with the synthesis method of the example 1 were analyzed by mass spectrometry for quantitative determination of the formed ester.

The instrument Massa quadrupolo LC massa, (Waters) 4 Ultima Platinum was used.

The used source was an electronspray source, positive and negative ions.

The two samples obtained from example 1 were dried in nitrogen flow and taken-up again in 500 microliters of acetonitrile; the injection volume was 200 microliters. Tuning for standards was performed: cholesteryl linoleate and 3α-diol (1-10 micrograms /ml).

On the basis of the molecular peak corresponding to molecular weight (555) it has been confirmed that the two samples obtained from example 1 resulted to be the ester of 3α-diol with linoleic acid.

### Example 6:

### Evaluation of effectiveness of ester according to the invention

For the experiments of effectiveness a test to evaluate the effect of the ester formed from linoleic acid obtained from example 1 and example 4 on acyl-coenzyme A:cholesterol acetyltransferase enzyme (ACAT) activity was performed. The experiment was performed in vitro according to a classic pharmacology method, used for the evaluation of the activity of this enzyme, reported in the method book: Drug discovery and evaluation, Pharmacological assay, Ed. H.G: Vogel (Springer), p.1119, 2002.

Hepatic microsomes were prepared as well as in the example 1.

Eight tubes were prepared, in all tubes linoleic acid-¹⁴C (10 microM) was incubated in presence of 200 micrograms of microsomes. Each tube contained phosphate buffer 0.04 M KH₂PO₄, pH 7.4 with 0.05 M KCl, 0.03 EDTA, 0.3 M sucrose, 1 mM α-cyclodextrin, final volume 750 microliters. The eight tubes have been incubated at 37°C for 10 min.

The esters of example 1 and 4 were pooled together and three sample solutions of concentration 12.5, 25 and 50 micromolar respectively were prepared in duplicate, therefore obtaining six sample solutions.

The six sample solutions were then transferred respectively in six of eight tubes prepared as above at the end of the 10 minutes (each concentration of ester separately incubated per tube). The seventh tube was added with ¹⁴C-linoleic acid alone (without esters) and the eighth with ethanol alone (carrier in which the esters were resuspended) to evaluate a possible effect of ethanol on the enzymatic activity. All the eight tubes have been incubated at 37°C for four minutes.

At the end of the time the reaction was stopped by adding chloroform:methanol 2:1. The samples of the eight tubes were extracted according to the Folch's method (1957). The lower phase of each sample containing the lipid extracts was separated and dried, then taken-up again in 1 ml of chloroform:methanol 2:1. The extract of each sample was dried and subjected to TLC.

A preparation of cholesteryl oleate and cholesteryl palmitoleate which run together in TLC was used as a standard and hexane:diethyleter:acetic acid 70:30:1.5 were used as solvents.

Spots were highlighted through iodine vapour. The bands which correspond to the standard for each TLC were taken off from the plate and counted in beta counter.

The obtained results (cpm) per sample were transformed in dpm/mg proteins and in inhibition percentage with reference to the untreated sample. The sample constituted by carrier alone resulted non-effective. An inhibition of 44% +/- 3.6 (mean +/- standard error) for the 25 microM concentration resulted, the effect resulted to be lower at concentrations 50 and 12.5 microM that is, 28%+/-1.9 and 2.5+/-2, respectively.

As evident from the above example, monoesters according to the invention are capable to reduce the accumulation of cholesterol esters by reducing the ACAT enzyme effect and indirectly intervene on β-oxidation in peroxisomes, a mechanism involved in the accumulation of very long chain saturated fatty acids, which are responsible for severe clinical symptoms, particularly for adrenoleucodystrophy. Thus, the use of the medicament according to the invention is extremely advantageous for treatment of pathologies involving a lipid metabolism alteration, for instance adrenoleucodystrophy, Alzheimer's disease and treatment of arteriosclerosis.

## Claims

1. A monoester of a steroid of formula I: 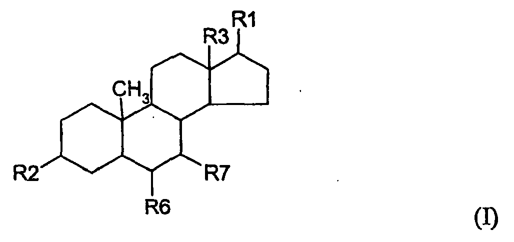 wherein
R1 and R2 are independently -OH or =O;
R3 is -CH₃;
R6 and R7 are independently -H or -OH;
with either a saturated or an unsaturated C₁₈-C₂₄ fatty acid for use as a medicament.

2. The monoester for use according to claim 1 wherein the monoester is formed between the steroid and the fatty acid at either the position 3 or the position 17 of steroid of formula I.

3. The monoester for use according to any one of claims 1-2, wherein the saturated or unsaturated fatty acid is a C₁₈-C₂₂ fatty acid.

4. The monoester for use according to claim 1, wherein the fatty acid is selected from the group consisting of oleic acid, stearic acid, elaidic acid, vaccenic acid, linoleic acid, conjugated linoleic acid, linolenic acid, α -linolenic acid, γ-linolenic acid, homo-gamma-linolenic acid, eleostearic acid, arachidonic acid, adrenic acid, erucic acid, nervonic acid, docosapentaenoic acid, eicosatetraenoic acid, eicosapentaenoic acid.

5. The monoester for use according to claim 4, wherein the fatty acid is an acid selected from the group consisting of linoleic acid, coniugated linoleic acid, linolenic acid, arachidonic acid and isomers thereof.

6. The monoester for use according to claim 5, wherein the fatty acid is linoleic acid or coniugated linoleic acid.

7. The monoester for use according to any one of claims 1-6, wherein the steroid of formula I is selected from the group consisting of 5α-androstan-17β-ol-3-one (DHT), 5α-androstan-3α,17β-diol (3α-diol), 5α-androstan-3β,17β-diol (3β-diol), 5α-androstan-3β,6α,17β-triol (6α-triol), 5α-androstan-3β,7β,17β-triol (7β-triol), 5α-androstan-3β,7α,17β-triol (7α-triol), 5α-androstan-3,17-dione (androstandione) and 5α-androstan-3α-ol-17-one (androsterone).

8. The monoester for use of claim 7 wherein the steroid of formula I is 5α-androstan-3α,17β-diol (3α-diol).

9. A monoester of the steroid of 5α-androstan-3α,17β-diol (3α-diol) with a C₁₈-C₂₄ fatty acid.

10. The monoester according to claim 9, wherein the fatty acid is selected from the group consisting of linoleic acid, conjugated linoleic acid, linolenic acid, arachidonic acid and isomers thereof.

11. The monoester according to claim 10, wherein the fatty acid is linoleic acid (*cis*,*cis*-9,12-octadecadienoic).

12. The monoester according to any one of claims 9 to 11 for use as a medicament.

13. A pharmaceutical composition comprising one or more monoesters according to any one of claims 1 to 8 and 12 and a pharmaceutically acceptable carrier.

14. Use of a monoester of a steroid of formula I: 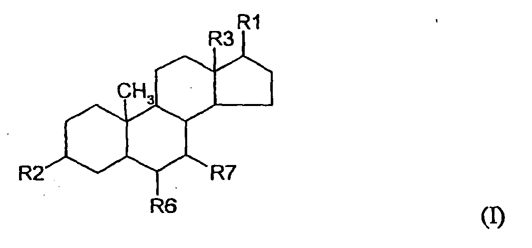 wherein
R1 and R2 are independently -OH or =O;
R3 is -CH₃;
R6 and R7 are independently -H or -OH;
with either a saturated or an unsaturated C₁₆-C₂₄ fatty acid or
of a monoester according to anyone of claims 9-11
for the manufacture of a medicament for treating a disease that shows abnormal accumulation of cellular and circulating cholesterol esters, said disease being selected from the group consisting of adrenoleucodystrophy, Alzheimer's disease and arteriosclerosis.

## Patentansprüche

1. Monoester eines Steroids der Formel (I): 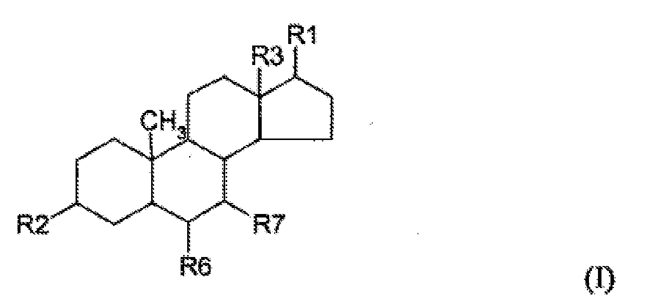 worin
R1 und R2 unabhängig voneinander für -OH oder =O stehen;
R3 für -CH₃ steht;
R6 und R7 unabhängig voneinander für -H oder -OH stehen;
mit entweder einer gesättigten oder ungesättigten C₁₈-C₂₄-Fettsäure zur Verwendung als Medikament.

2. Monoester zur Verwendung gemäß Anspruch 1, worin der Monoester gebildet wird zwischen dem Steroid und der Fettsäure entweder an der Position 3 oder an der Position 17 des Steroids der Formel (I).

3. Monoester zur Verwendung nach irgendeinem der Ansprüche 1 bis 2, worin die gesättigte oder ungesättigte Fettsäure eine C₁₈-C₂₂-Fettsäure ist.

4. Monoester zur Verwendung nach Anspruch 1, worin die Fettsäure gewählt ist aus der Gruppe, die besteht aus Ölsäure, Stearinsäure, Elaidinsäure, Vaccensäure, Linolsäure, konjugierte Linolsäure, Linolensäure, α-Linolensäure, γ-Linolensäure, homo-gamma-Linolensäure, Eleostearinsäure, Arachidonsäure, Adrensäure, Erucasäure, Nervoninsäure, Docosapentaensäure, Eicosatetraensäure, Eicosapentaensäure.

5. Monoester zur Verwendung nach Anspruch 4, worin die Fettsäure eine Säure ist, die gewählt ist aus der Gruppe, die besteht aus Linolsäure, konjugierter Linolsäure, Linolensäure, Arachidonsäure und Isomeren davon.

6. Monoester zur Verwendung nach Anspruch 5, worin die Fettsäure Linolsäure oder konjugierte Linolsäure ist.

7. Monoester zur Verwendung nach irgendeinem der Ansprüche 1 bis 6, worin das Steroid der Formel (I) gewählt ist aus der Gruppe, die besteht aus 5α-Androstan-17β-ol-3-on (DHT), 5α-Androstan-3α,17β-diol (3α-diol), 5α-Androstan-3β,17β-diol (3β-diol), 5α-Androstan-3β,6α,17β-triol (6α-triol), 5α-Androstan-3β,7β,17β-triol (7β-triol), 5α-Androstan-3β,7α,17β-triol (7α-triol), 5α-Androstan-3,17-dion (Androstandion) und 5α-Androstan-3α-ol-17-on (Androsteron).

8. Monoester zur Verwendung nach Anspruch 7, worin das Steroid der Formel (I) 5α-Androstan-3α,17β-diol (3α-diol) ist.

9. Monoester des Steroids 5α-Androstan-3α,17β-diol (3α-diol) mit einer C₁₈-C₂₄-Fettsäure.

10. Monoester nach Anspruch 9, worin die Fettsäure gewählt ist aus der Gruppe, die besteht aus Linolsäure, konjugierter Linolsäure, Linolensäure, Arachidonsäure und Isomeren davon.

11. Monoester nach Anspruch 10, worin die Fettsäure Linolsäure ist (cis, cis-9,12-Octadecadienensäure).

12. Monoester nach irgendeinem der Ansprüche 9 bis 11 zur Verwendung als Medikament.

13. Pharmazeutische Zusammensetzung, umfassend einen oder mehrere Monoester nach irgendeinem der Ansprüche 1 bis 8 und 12 und einen pharmazeutisch annehmbaren Träger.

14. Verwendung eines Monoesters eines Steroids der Formel (I): 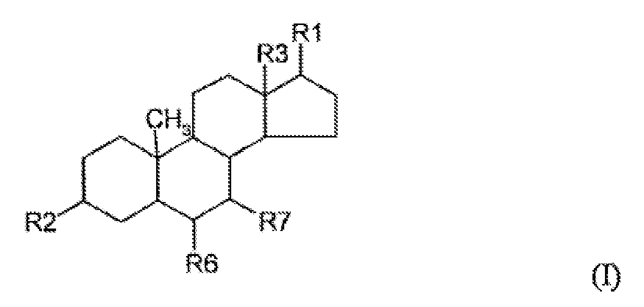 worin
R1 und R2 unabhängig voneinander für -OH oder =O stehen;
R3 für -CH₃ steht;
R6 und R7 unabhängig voneinander für -H oder -OH stehen;
mit entweder einer gesättigten oder ungesättigten C₁₆-C₂₄-Fettsäure oder eines Monoesters gemäß irgendeinem der Ansprüche 9 bis 11 für die Herstellung eines Medikaments zur Behandlung einer Krankheit, die eine anormale Ansammlung zellulärer oder im Kreislauf zirkulierender Cholesterolester zeigt, wobei die Krankheit gewählt ist aus der Gruppe, die gewählt ist aus Adrenoleucodystrophie, Alzheimer'scher Krankheit und Arteriosklerose.

## Revendications

1. Monoester d'un stéroïde de formule I : 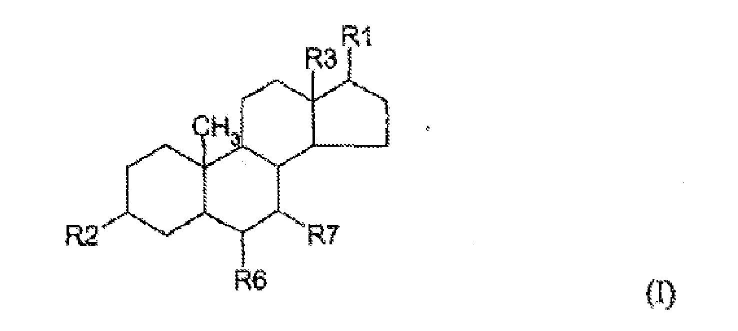 dans laquelle
R1 et R2 sont indépendamment -OH ou =O ;
R3 est -CH₃ ;
R6 et R7 sont indépendamment -H ou -OH ;
avec un acide gras C₁₈-C₂₄ saturé ou non
utilisable en tant que médicament.

2. Monoester utilisable selon la revendication 1, dans lequel le monoester est formé entre le stéroïde et l'acide gras soit à la position 3, soit à la position 17 du stéroïde de formule I.

3. Monoester utilisable selon l'une quelconque des revendications 1 ou 2, dans lequel l'acide gras saturé ou non est un acide gras C₁₈-C₂₂.

4. Monoester utilisable selon la revendication 1, dans lequel l'acide gras est choisi dans le groupe constitué par l'acide oléique, l'acide stéarique, l'acide élaïdique, l'acide vaccénique, l'acide linoléique, l'acide linoléique conjugué, l'acide linolénique, l'acide α-linoléique, l'acide γ-linolénique, l'acide homo-gamma-linolénique, l'acide éléostéarique, l'acide arachidonique, l'acide adrénique, l'acide érucique, l'acide nervonique, l'acide docosapentaénoïque, l'acide eicosatétraénoïque, et l'acide eicosapentaénoïque.

5. Monoester utilisable selon la revendication 4, dans lequel l'acide gras est un acide choisi dans le groupe constitué par l'acide linoléique, l'acide linoléique conjugué, l'acide linolénique, l'acide arachidonique et leurs isomères.

6. Monoester utilisable selon la revendication 5, dans lequel l'acide gras est l'acide linoléique ou l'acide linoléique conjugué.

7. Monoester utilisable selon l'une quelconque des revendications 1 à 6, dans lequel le stéroïde de formule I est choisi dans le groupe constitué par la 5α-androstan-17β-ol-3-one (DHT), le 5α-androstan-3α,17β-diol (3α-diol), le 5α-androstan-3β,17β-diol (3β-diol), le 5α-androstan-3β,6α,17β-triol (6α-triol), le 5α-androstan-3β,7β,17β-triol (7β-triol), le 5α-androstan-3β,7α,17β-triol (7α-triol), la 5α-androstan-3,17-dione (androstandione) et la 5α-androstan-3α-ol-17-one (androstérone).

8. Monoester utilisable selon la revendication 7, dans lequel le stéroïde de formule I est le 5α-androstan-3α,17β-diol (3α-diol).

9. Monoester du stéroïde de 5α-androstan-3α,17β-diol (3α-diol) avec un acide gras C₁₈-C₂₄.

10. Monoester selon la revendication 9, dans lequel l'acide gras est choisi dans le groupe constitué par l'acide linoléique, l'acide linoléique conjugué, l'acide linolénique, l'acide arachidonique et leurs isomères.

11. Monoester selon la revendication 10, dans lequel l'acide gras est l'acide linoléique (*cis,cis*-9,12-octadécadiénoïque).

12. Monoester selon l'une quelconque des revendications 9 à 11 utilisable à titre de médicament.

13. Composition pharmaceutique comprenant un ou plusieurs monoesters selon l'une quelconque des revendications 1 à 8 et 12 et un véhicule pharmaceutiquement acceptable.

14. Utilisation d'un monoester d'un stéroïde de formule I : 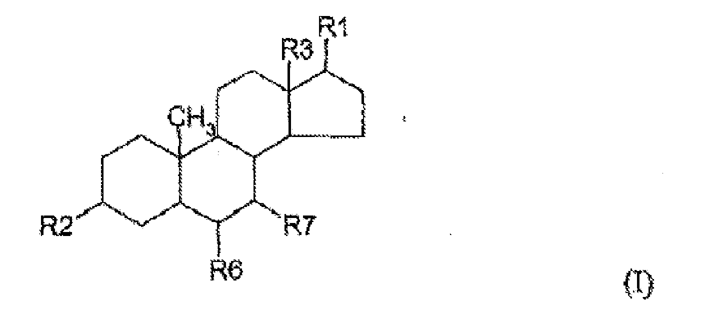 dans laquelle
R1 et R2 sont indépendamment -OH ou =O ; R3 est -CH₃ ;
R6 et R7 sont indépendamment -H ou -OH ; avec un acide gras C₁₆-C₂₄ saturé ou non ou
d'un monoester selon l'une quelconque des revendications 9 à 11
dans la fabrication d'un médicament destiné à traiter une maladie qui présente une accumulation anormale d'esters de cholestérol cellulaires et circulants, ladite maladie étant choisie dans le groupe constitué par l'adrénoleucodystrophie, la maladie d'Alzheimer et l'artériosclérose.
